# EUROPEAN PATENT APPLICATION

(11) **EP 1 837 653 A1**
(43) Date of publication of application: **26.09.2007**
(21) Application number: 07104437.4
(22) Date of filing: 19.03.2007
(51) Int. Cl.: G01N 33/18, G01N 31/22, C07C 309/11, C07C 309/46

(54) **Composition for measuring concentration of residual chlorine**

(30) Priority: 23.03.2006 JP 2006079839
(71) Applicant: MIURA CO., LTD., Matsuyama-shi Ehime 799-2696 (JP)
(72) Inventor: Mitsumoto, Hiroyuki, Ehime 799-2696 (JP)
(74) Representative: Marchant, James Ian

(57) **Abstract**

The present invention provides a composition for measuring residual chlorine concentration in the sample water, which is an aqueous solution comprising a specific benzidine compound, e.g., N,N'-bis(2-sulfoethyl)-3,3'-dimethylbenzidine disodium salt, and a surfactant, wherein the content of the surfactant is set to be 0.5% by weight or more and to be 0.5 weight-fold or more of the content of the benzidine compound, and the pH is adjusted to be in the acidic region.

This composition can prevent the benzidine compound from crystallizing in the aqueous solution under the low temperature condition such as in winter season or the like.

## Description

### Technical Field

The present invention relates to a composition for measuring residual chlorine concentration in a sample water, and specifically, to a one-solution type composition for measuring residual chlorine concentration in a sample water using a color reaction.

### Background Art

Daily life water such as tap water and well water, or swimming pool water has a chlorine agent such as sodium hypochlorite added thereto. Such a chlorine agent has effects such as disinfection and sterilization by oxidation. However, when there are suspensions, organic substances, metal ions, or the like in water, those effects may be deteriorated by having these substances react with the chlorine agent. Also, the effect of the chlorine agent may be decreased over time by having the chlorine agent in an open system such as a water storage tank or a swimming pool be diffused into the air. Therefore, it is necessary to measure the concentration of residual chlorine in water added with a chlorine agent regularly and confirm whether a required concentration thereof is maintained or not.

Further, with respect to the water treatment system using various filtration membranes such as a microfiltration membrane, an ultrafiltration membrane, a reverse osmosis membrane and a nanofiltration membrane, the filtration membrane is easily deteriorated by oxidation when a chlorine agent exists in the feed water. When the filtration membrane deteriorates, the quality of the treated water is deteriorated. Thus, in such the water treatment system, in general, an activated carbon filtration apparatus or sodium bisulfite (SBS) addition apparatus is installed on the upper side of the filtration membrane, and the chlorine agent is removed from the feed water. In this case, it is necessary to measure the concentration of residual chlorine in the feed water, which was passed through the activated carbon filtration apparatus or added with sodium bisulfite, regularly to confirm whether the chlorine agent is completely removed or not.

A method of measuring the concentration of residual chlorine in water using a color reagent such as o-tolidine or N,N-diethylphenylene diamine (DPD) has been widely used. Moreover, recently, a method of measuring using a benzidine compound that is more excellent in stability than DPD as a color reagent is suggested. For example, JP-A Nos. 2002-350416 and 9-133671 describe the use of a dialkylbenzidine compound or a tetraalkylbenzidine compound as a color reagent.

For measurement of the concentration of residual chlorine at the field, generally a portable measurement device or a simplified measurement kit is used in many cases. However, since such the portable measurement device or the simplified measurement kit requires proficiency in the operation and the operation itself becomes a troublesome as the measurement frequency increases, a demand for an automated measurement device also increases. The automated measurement device is constituted such that a series of operations such as a step of sampling the sample water, a step of adding a color reagent and a step of measuring the concentration of residual chlorine are carried out automatically, thus it is possible to carry out continuous measurement.

The automated measurement device usually has a color reagent stored within the device as a chemical solution state such as an aqueous solution, and it is constituted such that during the step of adding the color reagent, a predetermined amount of the chemical solution is added to the sample water. When the chemical solution becomes short from the consumption, a new chemical solution is supplemented. Such the automated measurement device is installed in various places depending on the water system to be observed, but since it is seldom installed in the place where the temperature control is performed, the chemical solution, which does not degraded in the hostile temperature condition, for example, in a wide range from 5 to 50°C is required. However, the color reagent used in measuring the concentration of residual chlorine, for example, is in an aqueous solution, and its solubility decreases as the temperature decreases. Therefore, when the surrounding temperature is lowered to around 5 to 10°C in the winter season or the like, block is generated in the supply path of the chemical solution and the like by crystallization of the color reagent in the chemical solution, thus there is a problem that a predetermined amount of the chemical solution cannot be properly added to the sample water during the step of adding the color reagent. Moreover, even when a predetermined amount of the chemical solution is able to be added to the sample water, there is a problem that the measurement lacks reliability, since the concentration of the color reagent in the chemical solution is changed.

It is an object of the present invention to provide a composition for measuring residual chlorine concentration that can prevent the color reagent from crystallization in a chemical solution. Specifically, it is an object of the present invention to provide a composition for measuring residual chlorine concentration that can prevent the color reagent from crystallization in a chemical solution even when stored at a low temperature, for example, at around 5°C for a long time.

### Disclosure of the Invention

The composition for measuring residual chlorine concentration of the present invention is used to measure the concentration of residual chlorine in the sample water, wherein the composition is an aqueous solution comprising a benzidine compound represented by the following formula (I) and a surfactant, wherein the content of the surfactant is set to be 0.5% by weight or more and to be 0.5 weight-foldor more of the content of the benzidine compound, and the pH is adjusted to be in the acidic region. 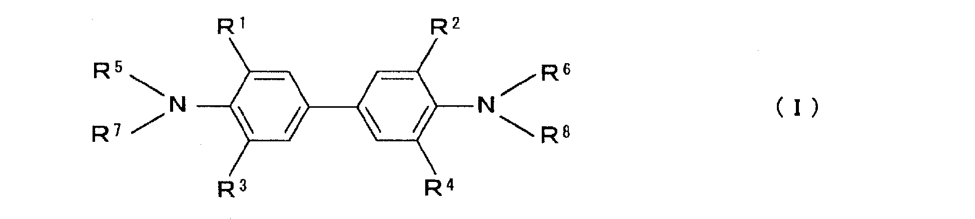 wherein, R¹ and R² are each independently an alkyl group having 1 to 6 carbon atoms; and R³ and R⁴ are each independently an alkyl group having 1 to 6 carbon atoms, or hydrogen atoms at the same time. R⁵ and R⁶ are each independently a hydrocarbon group having 1 to 8 carbon atoms, or hydrogen atoms at the same time. R⁷ and R⁸ are each independently a hydrogen atom, a sulfohydrocarbon group having 1 to 8 carbon atoms which may form an alkali metal salt having one or more hydroxyl group or a carboxyhydrocarbon group having 1 to 8 carbon atoms which may form an alkali metal salt having one or more hydroxyl group, but R7 and R8 are not be a hydrogen atom at the same time.

The composition for measuring residual chlorine concentration of the invention can prevent the benzidine compound from crystallizing in the aqueous solution under the low temperature condition such as in winter season or the like. For example, the composition can increase the solubility of the benzidine compound as a color reagent by the function of a surfactant even when stored under the temperature condition of 5°C for a long time, thus preventing crystallization in the aqueous solution. From this, when the composition for measuring residual chlorine concentration of the present invention is used in an automated device for measuring the concentration of residual chlorine, a predetermined amount of the composition can be added stably to the sample water and the concentration of the color reagent in the chemical solution can be maintained constant, thereby establishing the reliability in the measured value of the residual chlorine concentration.

The composition for measuring residual chlorine concentration is usually added with an acid to adjust the pH of the composition to the acidic region. In this case, the composition may be added with an alkali metal salt of an acid to precisely adjust the pH of the composition to the acidic region. Examples of the acid and the alkali metal salt of the acid used herein include phosphoric acid and sodium phosphate, respectively.

Moreover, the composition for measuring residual chlorine concentration may further contain a dye for coloring the aqueous solution in a color development wavelength region other than the color development wavelength region derived from the reaction of the benzidine compound and the residual chlorine.

Examples of the benzidine compound used in the composition for measuring residual chlorine concentration include an N,N'-bis(2-sulfoethyl)-3,3'-dimethylbenzidine disodium salt. The surfactant is usually at least one selected from the group consisting of a nonionic surfactant and an anionic surfactant.

### Best Mode for Carrying Out the Invention

The composition for measuring the concentration of residual chlorine of the present invention (hereinafter, simply referred to as "the composition") is a one-package aqueous solution used for optically measuring the concentration of residual chlorine in the sample water, which comprises an aqueous solution containing a specific benzidine compound, and a surfactant.

The benzidine compound as used herein are an oxidative chromogenic color reagent developing to yellow to blue green color, which shows the absorption peaks at a wavelength of around 360 to 380 nm, around 450 to 470 nm, and around 640 to 660 nm due to the reaction of the residual chlorine reacts in the acidic region, and it is represented by the formula (I). 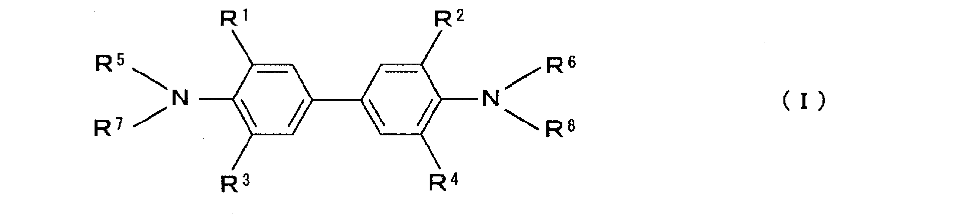 wherein, R¹ and R² are each independently an alkyl group having 1 to 6 carbon atoms; and R³ and R⁴ are each independently an alkyl group having 1 to 6 carbon atoms, or hydrogen atoms at the same time. Accordingly, the benzidine compound represented by the formula (I) is a dialkylbenzidine compound (specifically 3,3'-dialkylbenzidine compound, and a tetraalkylbenzidine compound (specifically 3,3',5,5'-tetraalkylbenzidine compound).

R⁵ and R⁶ are each independently a hydrocarbon group having 1 to 8 carbon atoms, or hydrogen atoms at the same time. R⁷ and R⁸ are each independently a hydrogen atom, a sulfohydrocarbon group having 1 to 8 carbon atoms which may form an alkali metal salt having one or more hydroxyl group or a carboxyhydrocarbon group having 1 to 8 carbon atoms which may form an alkali metal salt having one or more hydroxyl group, but R⁷ and R⁸ are not be a hydrogen atom at the same time.

Examples of the alkyl group having 1 to 6 carbon atoms represented by the R¹ and R² include a methyl group, an ethyl group, a propyl group, an isopropyl group, a butyl group, an isobutyl group, a sec-butyl group, a tert-butyl group, a pentyl group, a 1-methylbutyl group, a 2-methylbutyl group, a 3-methylbutyl group, a 1-ethylpropyl group, a tert-pentyl group, a 1,2-diethylpropyl group, hexyl group and the like. In the case of the dialkylbenzidine compound, it is preferable that any one of R¹ and R² is a methyl group, and it is more preferable that both R¹ and R² are a methyl group. Further, in the case of the tetraalkylbenzidine compound, it is preferable that both R¹ and R² are a methyl group or an ethyl group.

In the case where the benzidine compound is the tetraalkylbenzidine compound, examples of the alkyl group having 1 to 6 carbon atoms represented by R³ and R⁴ include the same examples as for the above-mentioned R¹ and R². It is preferable that R³ and R⁴ are a methyl group is or an ethyl group.

Examples of the hydrocarbon group having 1 to 8 carbon atoms represented by R⁵ and R⁶ include the same examples as for the above-mentioned R¹ and R², a benzyl group, a phenyl group, an alkylphenyl group and the like. For the formula (I), it is preferable that R⁵ and R⁶ is each independently a hydrogen atom, or both R⁵ and R⁶ are a methyl group or an ethyl group, particularly an ethyl group.

The sulfohydrocarbon group having 1 to 8 carbon atoms which may form an alkali metal salt having one or more hydroxyl group represented by R⁷ and R⁸ refers to a group in which the hydrocarbon group is substituted with a sulfonic acid group, as in the above-mentioned R⁵ and R⁶. The substitution position of the sulfonic acid group is not particularly limited, but it is generally substituted to the end of an alkyl group, a benzyl group or a phenyl group is preferred. Further, if the sulfohydrocarbon group has one or more hydroxyl group, the substitution position and the number of the hydroxyl group are not particularly limited. Preferable examples of the sulfohydrocarbon group include a 2-sulfoethyl group, a 3-sulfopropyl group, a 2-hydroxy-3-sulfopropyl group, a 2-hydroxy-2-sulfoethyl group, a 4-sulfobutyl group, and a 2,4-disulfobenzyl group.

The carboxyhydrocarbon group having 1 to 8 carbon atoms which may have one or more hydroxyl group represented by R⁷ and R⁸ refers to a group in which the hydrocarbon group is substituted with a carboxyl group, as in the above-mentioned R⁵ and R⁶. The substitution position of the carboxyl group is not particularly limited, but it is generally substituted to the end of an alkyl group, a benzyl group or a phenyl group is preferred. Further, if the carboxyhydrocarbon group has one or more hydroxyl group, the substitution position and the number of the hydroxyl group are not particularly limited. In this embodiment, preferable examples of the carboxyhydrocarbon group include a 2-carboxyethyl group, a 3-carboxypropyl group, a 2-hydroxy-3-carboxypropyl group, a 2-hydroxy-2-carboxyethyl group, a 4-carboxybutyl group, and a 2,4-dicarboxybenzyl group.

Preferable examples of the dialkylbenzidine compounds among the benzidine compounds represented by the formula (I) include N,N'-bis(2-sulfoethyl)-3,3'-dimethylbenzidine; N,N'-bis(3-sulfopropyl)-3,3'-dimethylbenzidine; N,N'-bis(2-hydroxy-2-sulfoethyl)-3,3'-dimethylbenzidine; N,N'-bis(2-hydroxy-3-sulfopropyl)-3,3'-dimethylbenzidine; N,N'-bis(4-sulfobutyl)-3,3'-dimethylbenzidine; N,N'-bis(3-sulfopropyl)-N,N'-diethyl-3,3'-dimethylbenzidine; N,N'-bis(2,4-disulfobenzyl)-3,3'-dimethylbenzidine; and alkali metal salts thereof. Among these compounds, those in a form of sodium salt are particularly preferably used due to their high water solubility and difficulty in causing crystallization at ambient temperatures.

Preferable examples of tetraalkylbenzidine compounds represented by the formula (I) include N- (2-sulfoethyl)-3,3',5,5'-tetramethylbenzidine; N-(3-sulfopropyl)-3,3',5,5'-tetramethylbenzidine; N-(4-sulfobutyl)-3,3',5,5'-tetramethylbenzidine; N-(2-hydroxy-2-sulfoethyl)-3,3',5,5'-tetramethylbenzidine; N-(2-hydroxy-3-sulfopropyl)-3,3',5,5'-tetramethylbenzidine; N-(2,4-disulfobenzyl)-3,3',5,5'-tetramethylbenzidine; N,N'-bis(2-sulfoethyl)-3,3',5,5'-tetramethylbenzidine; N,N'-bis(3-sulfopropyl)-3,3',5,5'-tetramethylbenzidine; N,N'-bis(4-sulfobutyl)-3,3',5,5'-tetramethylbenzidine; N,N'-bis(2-hydroxy-2-sulfoethyl)-3,3',5,5'-tetramethylbenzidine and N,N'-bis(2-hydroxy-3-sulfopropyl)-3,3',5,5'-tetramethylbenzidine; N,N'-bis(2,4-disulfobenzyl)-3,3',5,5'-tetramethylbenzidine; and alkali metal salts thereof; and the like. Among these compounds, the compounds in the forms of sodium salt are particularly preferable due to their high water solubility and difficulty in causing crystallization at ambient temperatures.

Herein, the benzidine compounds are usually used alone, but they can be used in a mixture of two or more kinds. If they are used in a mixture of two or more kinds, the two kinds selected from the dialkylbenzidine compounds may be used, or the two kinds selected from the tetraalkylbenzidine compounds may be used. Alternatively, the dialkylbenzidine compounds and the tetraalkylbenzidine compounds may be used in combination.

The content of the benzidine compound in the composition of the invention is not particularly limited, but it is usually determined based on the amount of the obtained sample water, the maximum value of the residual chlorine concentration which can be measured and the amount of the composition added to the sample water. That is, when a predetermined amount of the composition is added to the sample water containing an amount of residual chlorine calculated from the product of the amount of the obtained sample water and the maximum value of the residual chlorine concentration which can be measured, an amount of the benzidine compound and the residual chlorine capable of carrying out equivalent reaction is combined. As an example of combining N,N'-bis(2-hydroxy-3-sulfopropyl)-3,3'-dimethylbenzidine disodium salt as the benzidine compound, when the amount of the obtained sample water measured is 4 mL, the maximum value of the residual chlorine concentration which can be measured is 2.5 mg/L (in terms of C12) and the amount of the composition added to the sample water is 60 µL, the content of the benzidine compound is set to be 0.5% by weight.

The surfactant used in the composition of the invention is a surfactant which prevents the benzidine compound from crystallization in the composition by increasing solubility of the benzidine compound at a low temperature. As the surfactant, a nonionic surfactant or an anionic surfactant is preferably used in the viewpoint that the critical micelle concentration (CMC) is relatively low, and that the surfactant does not generate insoluble suspension by combination with organic substances in the sample water. Examples of the usable nonionic surfactant include polyoxyethylene alkyl ether, polyoxyethylene alkylphenyl ether, polyoxyethylene distyrenated phenyl ether and the like, and higher alcoholic, nonionic surfactants. Also, examples of the usable anionic surfactant include alkyldiphenyl ether disulfonate and the like.

For the composition of the invention, the content of the surfactant is 0.5% by weight or more, preferably 0.5 to 5.0% by weight, and also is 0.5 weight-fold or more of the content of the benzidine compound. When the content of the surfactant is less than 0.5% by weight, the surfactant can hardly work to prevent a formation of crystals. In addition, when the content of the surfactant is less than 0.5 weight-fold of that of the benzidine compound, since crystals are easily formed in short time when the temperature of the composition is lowered to a lower temperature, for example, 5°C, there is a possibility of blockage in the chemical solution supply path or variation in an automated measurement device for residual chlorine, or the concentration of the benzidine compound in the composition added to the sample water becomes unstable.

The pH of the composition of the invention is adjusted to in the acidic region, preferably 3.5 or lower, more preferably 3.0 or lower, and particularly preferably 1.9 or lower. By such adjustment, even when the composition of the invention is stored at a temperature condition of 5 to 50°C for a long time, temporal elevation of the absorbance in the maximum absorption wavelength area during coloration is inhibited upon color development by the reaction of the benzidine compound and the residual chlorine, and the measurement of the stabilized residual chlorine concentration can be carried out.

In the composition of the invention, if necessary, a dye in a predetermined color can be contained to optically determine whether the composition is properly added or not when added to the sample water. The benzidine compound are compounds which do not show absorption (color development) when the concentration of residual chlorine is zero. Therefore, even when the concentration of residual chlorine is zero by containing a dye in the composition, the addition state of the composition to the sample water can be confirmed based on the degree of coloration of the sample water. The dye as used herein is not particularly limited as long as it performs color development of the composition, that is, the aqueous solution in a color development wavelength region other than the color development wavelength region derived from the reaction of the benzidine compound and the residual chlorine. For example, if the color development wavelength region derived from the reaction of the benzidine compound and the residual chlorine is set around 640 to 660 nm, a synthetic dye such as New Coccin (CI Acid Red 18) and Acid Red (CI Acid Red 52) can be used.

The content of the dye in the composition is not particularly limited, but usually the content in the range of 0.01 to 0.50% by weight is suitably set in the viewpoint of colorability, solubility and economic efficiency.

The composition of the invention can be prepared by adding the benzidine compound, a surfactant and, if necessary, a dye to water, homogeneously mixing them, and adjusting the pH. For example, the composition of the invention is prepared by dissolving the benzidine compound and a surfactant to water having its pH adjusted by preliminarily adding an acid, and if necessary, adding a dye thereto.

The type of water as used herein is usually distilled water. The acid which can be used for adjusting the pH is an inorganic acid or an organic acid. These acids can be used alone or in suitable combinations thereof. Examples of the usable inorganic acid include an acid which does not have oxidative property or reductive property, such as sulfuric acid, hydrochloric acid, phosphoric acid and the like. Examples of the usable organic acid include citric acid, acetic acid, succinic acid, oxalic acid and the like.

If necessary, an alkali metal salt of an acid can be added to precisely adjust the pH of the composition. Examples of the usable alkali metal salt of an acid include an alkali metal salt of phosphoric acid such as trisodium phosphate and sodium phosphate, and an alkali metal salt of citric acid such as sodium citrate. In the case of setting the pH of the composition to be 1.0 or lower, e.g., 0.6, the desired pH can be simply obtained by first preparing the composition with a pH of less than 0.6 using sulfuric acid and phosphoric acid, and then adding trisodium phosphate.

Herein, when phosphoric acid or an alkali metal salt of phosphoric acid is used in the composition for adjusting the pH of the composition in the acidic region, they may form a complex with metal ions, which interfere with the measurement of residual chlorine, including reducing metal ions such as Fe2+, or oxidizing metal ions such as Cr⁺⁶ and Fe⁺³ in the sample water to mask such metal ions.

The composition of the invention is a one-package aqueous solution, which can be used for measuring residual chlorine concentration in water. The type of water containing residual chlorine which is a subject to be measured for the residual chlorine concentration is not particularly limited, but it can be widely applied, for example, to daily life water, swimming pool water, industrial water or industrial process water.

When measuring the concentration of residual chlorine, the measurement is carried out after pre-setting the measurement conditions such as the concentration of the benzidine compound contained in the composition, the amount of the obtained sample water, the maximum value of the residual chlorine concentration which can be measured and the amount of the composition added in the sample water. First, in the measurement process, the composition is added to the sample water obtained from the water system in the observation such that the benzidine compound is contained in the equivalent amount or more of the residual chlorine amount calculated from the product of the amount of the obtained sample water and the maximum value of the residual chlorine concentration. That is, when adding the composition to the sample water, the benzidine compound is controlled such that it is contained in the sample water in an amount to cover the predetermined range which can be measured. Next, with respect to the sample water containing the composition, the transmission (or absorbance) near the maximum absorption wavelength (e.g., 655 nm where commercially available red LED can be used, or 470 nm where commercially available blue LED can be used) when the color development is performed by the reaction of the benzidine compound and the residual chlorine is detected. Then, the concentration of residual chlorine in the sample water is calculated based on the calibration curve showing the relationship between the pre-calculated residual chlorine concentration and the transmission (or absorbance).

As described in the above, according to the invention, a composition for measuring residual chlorine that can prevent the benzidine compound from crystallization at a low temperature, in particular, at a temperature of 5 to 10°C can be established. That is, for the composition of the invention, under the low temperature condition such as the winter season, the benzidine compound can increase its solubility by the function of a surfactant, thereby crystallization in the chemical solution being prevented. As a result, for example, with respect to an automated device for measuring the concentration of residual chlorine, a predetermined amount of chemical solution can be added stably to the sample water and the concentration of the color reagent in the chemical solution can be maintained regularly, thus the reliability in the measured value is obtained.

### Examples

### Comparative Examples 1 to 7

Using N,N'-bis(2-hydroxy-3-sulfopropyl)-3,3'-dimethylbenzidine disodium salt (manufactured by Dojindo Laboratories; trade name: "SAT-3") as the benzidine compound which is a color reagent, a one-package aqueous solution was prepared by mixing various components in the combination ratio as shown in Table 1. In Table 1, polyoxyethylene alkylphenyl ether (manufactured by Wako Pure Chemical Industries, Ltd.; trade name: "polyoxyethylene (10) octylphenyl ether") (hereinafter, referred to as "POE") and higher alcoholic, nonionic surfactant (manufactured by Sanyo Chemical Industries, Ltd.; trade name "NAROACTY HN-100") were used as a nonionic surfactant, and sodium alkyldiphenyl ether disulfonate (manufactured by Sanyo Chemical Industries, Ltd.; trade name: "SANDET ALH") was used as an anionic surfactant. The "New Coccin" as depicted in Table 1 is an edible dye (CI Acid Red 18).

Next, 100 mL of the aqueous solution in each of Comparative Examples were stored in a freezer setting the temperature at 5°C, and the existence of crystals after 2 days, 7 days, 14 days, 30 days, 60 days and 120 days were observed. The results are presented in Table 2.

**Table 1**

| Composition | Combination ratio (wt%) | | | | | | |
|---|---|---|---|---|---|---|---|
| | Comp. Ex. 1 | Comp. Ex. 2 | Comp. Ex. 3 | Comp. Ex. 4 | Comp. Ex. 5 | Comp. Ex. 6 | Comp. Ex. 7 |
| N,N'-bis(2-hydroxy-3-sulfopropyl)-3,3'-dimethylbenzidine disodium salt | 1.0 | 0.5 | 2.5 | 0.5 | 2.5 | 0.5 | 2.5 |
| Sulfuric acid (47%) | 7.5 | 7.5 | 7.5 | 7.5 | 7.5 | 7.5 | 7.5 |
| Phosphoric acid | 5.0 | 5.0 | 5.0 | 5.0 | 5.0 | 5.0 | 5.0 |
| Trisodium phosphate•12H₂O | 4.0 | 4.0 | 4.0 | 4.0 | 4.0 | 4.0 | 4.0 |
| POE | - | 0.25 | 1.0 | - | - | - | - |
| NAROACTY HN-100 | - | - | - | 0.25 | 1.0 | - | - |
| SANDET ALH | - | - | - | - | - | 0.25 | 1.0 |
| New Coccin | 0.025 | 0.025 | 0.025 | 0.025 | 0.025 | 0.025 | 0.025 |
| Distilled water | 82.475 | 82.725 | 79.975 | 82.725 | 79.975 | 82.725 | 79.975 |

**Table 2**

| Elapsed days | Existence of crystals | | | | | | |
|---|---|---|---|---|---|---|---|
| | Comp. Ex. 1 | Comp. Ex. 2 | Comp. Ex. 3 | Comp. Ex. 4 | Comp. Ex. 5 | Comp. Ex. 6 | Comp. Ex. 7 |
| 2 days | No | No | No | No | No | No | No |
| 7 days | No | No | No | No | No | No | No |
| 14 days | Yes | Yes | Yes | Yes | Yes | Yes | Yes |
| 30 days | Yes | Yes | Yes | Yes | Yes | Yes | Yes |
| 60 days | Yes | Yes | Yes | Yes | Yes | Yes | Yes |
| 120 days | Yes | Yes | Yes | Yes | Yes | Yes | Yes |

### Examples 1 to 7

Using N,N'-bis(2-hydroxy-3-sulfopropyl)-3,3'-dimethylbenzidine disodium salt (manufactured by Dojindo Laboratories; trade name: "SAT-3") as the benzidine compound which is a color reagent, a one-package aqueous solution was prepared by mixing various components in the combination ratio as shown in Table 3. In Table 3, polyoxyethylene alkylphenyl ether (manufactured by Wako Pure Chemical Industries, Ltd.; trade name: "polyoxyethylene (10) octylphenyl ether") and higher alcoholic, nonionic surfactant (manufactured by Sanyo Chemical Industries, Ltd.; trade name "NAROACTY HN-100") were used as a nonionic surfactant, and sodium alkyldiphenyl ether disulfonate (manufactured by Sanyo Chemical Industries, Ltd.; trade name: "SANDET ALH") was used as an anionic surfactant. The "New Coccin" as depicted in Table 1 is an edible dye (CI Acid Red 18).

Next, 100 mL of the aqueous solution in each Examples were stored in a freezer setting the temperature at 5°C, and the existence of crystals after 2 days, 7 days, 14 days, 30 days, 60 days and 120 days were observed. The results are presented in Table 4.

**Table 3**

| Composition | Combination ratio (wt%) | | | | | | |
|---|---|---|---|---|---|---|---|
| | Example 1 | Example 2 | Example 3 | Example 4 | Example 5 | Example 6 | Example 7 |
| N,N'-bis(2-hydroxy-3-sulfopropyl)-3,3'-dimethylbenzidine disodium salt | 0.5 | 2.0 | 2.0 | 0.5 | 2.0 | 0.5 | 2.0 |
| Sulfuric acid (47%) | 7.5 | 7.5 | 7.5 | 7.5 | 7.5 | 7.5 | 7.5 |
| Phosphoric acid | 5.0 | 5.0 | 5.0 | 5.0 | 5.0 | 5.0 | 5.0 |
| Trisodium phosphate•12H₂O | 4.0 | 4.0 | 4.0 | 4.0 | 4.0 | 4.0 | 4.0 |
| POE | 0.5 | 1.0 | 5.0 | - | - | - | - |
| NAROACTY HN-100 | - | - | - | 0.5 | 1.0 | - | - |
| SANDET ALH | - | - | - | - | - | 0.5 | 1.0 |
| New Coccin | 0.025 | 0.025 | 0.025 | 0.025 | 0.025 | 0.025 | 0.025 |
| Distilled water | 82.475 | 80.475 | 76.475 | 82.475 | 80.475 | 82.475 | 80.475 |

**Table 4**

| Elapsed days | Existence of crystals | | | | | | |
|---|---|---|---|---|---|---|---|
| | Example | Example | Example | Example | Example | Example | Example |
| 2 days | No | No | No | No | No | No | No |
| 7 days | No | No | No | No | No | No | No |
| 14 days | No | No | No | No | No | No | No |
| 30 days | No | No | No | No | No | No | No |
| 60 days | No | No | No | No | No | No | No |
| 120 days | No | No | No | No | No | No | No |

### Evaluation

According to Table 2, each aqueous solution prepared such that the content of the surfactant is less than 0.5% by weight, or the content of the surfactant is less than 0.5 weight-fold of that of the benzidine compound formed color reagent crystals of the benzidine compound in short time of 7 to 14 days under the temperature condition of 5°C. Meanwhile, according to Table 4, each aqueous solution prepared such that the content of the surfactant is 0.5% by weight or more, and the content of the surfactant is 0.5 weight-fold or more of that of the benzidine compound did not show any crystal forming at the time of 120 elapsed days under the temperature condition of 5°C. Accordingly, when the content of the surfactant in the aqueous solution is 0.5% by weight or more and the content of the surfactant is 0.5 weight-fold or more of that of the benzidine compound, it is found that the crystallization of the benzidine compound at a low temperature can be prevented for a long time.

## Claims

1. A composition for measuring residual chlorine concentration in a water sample, which is an aqueous solution comprising a benzidine compound represented by the following formula (I) and a surfactant, **characterized in that**,
the content of the surfactant is 0.5% by weight or more and is 0.5 times by weight or more of the content of the benzidine compound, and the pH is adjusted to be in the acidic range: 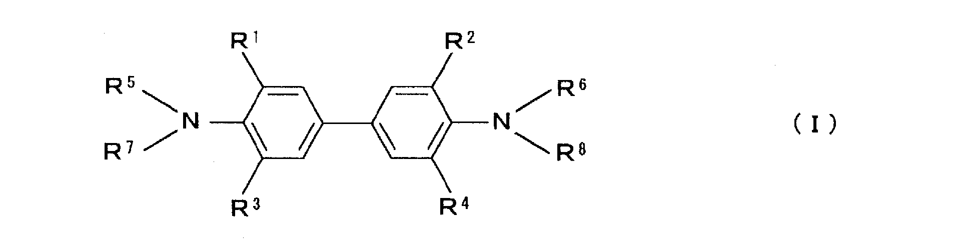 wherein, R¹ and R² are each independently an alkyl group having 1 to 6 carbon atoms; and R³ and R⁴ are each independently an alkyl group having 1 to 6 carbon atoms, or hydrogen atoms at the same time, R⁵ and R⁶ are each independently a hydrocarbon group having 1 to 8 carbon atoms, or hydrogen atoms at the same time, R⁷ and R⁸ are each independently a hydrogen atom, a sulfohydrocarbon group having 1 to 8 carbon atoms which may form an alkali metal salt having one or more hydroxyl groups or a carboxyhydrocarbon group having 1 to 8 carbon atoms which may form an alkali metal salt having one or more hydroxyl groups, with the proviso that R⁷ and R⁸ are not both a hydrogen atom.

2. A composition for measuring residual chlorine concentration according to claim 1, wherein an acid is added to adjust the pH to the acidic range.

3. A composition for measuring residual chlorine concentration according to claim 2, wherein an alkali metal salt of an acid is added to adjust precisely the pH to the acidic range.

4. A composition for measuring residual chlorine concentration according to claim 3, wherein the acid is phosphoric acid and the alkali metal salt of an acid is sodium phosphate.

5. A composition for measuring residual chlorine concentration according to any one of claims 1 to 4, which further comprises a dye for coloring the aqueous solution in a color development wavelength region other than the color development wavelength region derived from the reaction of the benzidine compound and residual chlorine.

6. A composition for measuring residual chlorine concentration according to any one of claims 1 to 5, wherein the benzidine compound is an N,N'-bis(2-sulfoethyl)-3,3'-dimethylbenzidine disodium salt.

7. A composition for measuring residual chlorine concentration according to any one of claims 1 to 6, wherein the surfactant is at least one surfactant selected from the group consisting of nonionic surfactants and anionic surfactants.
